# EUROPÄISCHE PATENTANMELDUNG

(11) **EP 3 693 735 A1**
(43) Veröffentlichungstag der Anmeldung: **12.08.2020**
(21) Anmeldenummer: 19155791.7
(22) Anmeldetag: 06.02.2019
(51) Int. Cl.: G01N 33/00, G01N 21/84

(54) **VERFAHREN UND VORRICHTUNG ZUR ANALYSE VON PFLANZEN**

(71) Anmelder: SpexAI GmbH, 01099 Dresden (DE)
(72) Erfinder: Niehaus, Ben, 01099 Dresden (DE)
(74) Vertreter: dompatent von Kreisler Selting Werner - Partnerschaft von Patent- und Rechtsanwälten mbB

(57) **Zusammenfassung**

Die vorliegende Erfindung betrifft ein Verfahren zur Analyse einer Pflanze (18), insbesondere zur Analyse von Cannabis, unter Verwendung einer Beleuchtungseinheit (12), einer Sensoreinheit (14) und einer Auswerteeinheit (16), wobei die Auswerteeinheit (16) einen datenbasierten Klassifikator (20). Zudem betrifft die vorliegende Erfindung eine Vorrichtung (10) zur Analyse einer Pflanze (18). Die Vorrichtung (10) umfasst eine Beleuchtungseinheit (12) zur Beleuchtung der zu analysierenden Pflanze und eine Sensoreinheit (14) zur Aufnahme von Analyse-Eingangsdaten, wobei die Analyse-Eingangsdaten zumindest spektrale Informationen, insbesondere ein Absorptionsspektrum oder ein Reflexionsspektrum der Trainings-Pflanze, enthalten. Zudem umfasst die Vorrichtung eine Auswerteeinheit (16) zur Auswertung der aufgenommenen Analyse-Eingangsdaten und zum Bestimmen mindestens einer Eigenschaft der zu analysierenden Pflanze. Dabei ist die Auswerteeinheit (16) dazu ausgelegt ist, die mindestens eine Eigenschaft der Pflanze unter Verwendung eines datenbasierten Klassifikators (20) sowie der zuvor aufgenommenen Analyse-Eingangsdaten zu bestimmen.

## Beschreibung

Die Erfindung betrifft ein Verfahren und eine Vorrichtung zur Analyse von Pflanzen. Insbesondere betrifft die Erfindung ein automatisiertes, optisches Verfahren zur Analyse und Bewertung von Pflanzeneigenschaften, insbesondere im Zusammenhang mit Cannabis.

Die Beurteilung der Wachstumsbedingungen von Pflanzen, sowohl im Feld als auch im Gewächshaus, obliegt auch heute noch weitgehend dem menschlichen Auge. Eine solche "manuelle" Analyse und Bewertung von Pflanzeneigenschaften ist jedoch zeitaufwendig, kostspielig und ungenau.

Beim Anbau von Pflanzen sind insbesondere der Gesundheitszustand bzw. ein möglicher Krankheitsbefall sowie die Stressoren von besonderer Bedeutung. Ist eine Pflanze etwa von Insekten, Pilzen oder Viren befallen, leiden sowohl Qualität als auch Quantität bei der späteren Ernte. Häufig ist ein Krankheitsbefall jedoch nur sehr spät für das menschliche Auge sichtbar.

Weitere Eigenschaften von Pflanzen, wie zum Beispiel der Wassergehalt oder etwa der THC-Gehalt von Cannabis, sind durch die manuelle Analyse besonders schwer zu erkennen.

Aufgabe der vorliegenden Erfindung ist es daher, ein Verfahren und eine Vorrichtung bereitzustellen, die es erlauben, die Eigenschaften einer Pflanze, insbesondere die Eigenschaften von Cannabis, bereits in einem frühen Stadium zu analysieren, um zuverlässige Aussagen über die zukünftig zu erwartenden Wachstumsbedingungen treffen zu können.

Zur Lösung der vorstehend genannten Aufgabe wird insbesondere ein Verfahren zur Analyse einer Pflanze, insbesondere zur Analyse von Cannabis, unter Verwendung einer Beleuchtungseinheit, einer Sensoreinheit und einer Auswerteeinheit vorgeschlagen, wobei die Auswerteeinheit einen datenbasierten Klassifikator enthält und das Verfahren die nachfolgenden Schritte aufweist:
- Trainieren des Klassifikators, mit den folgenden Schritten:
   - Beleuchten einer Trainings-Pflanze mit mindestens einer bekannten Eigenschaft unter Verwendung der Beleuchtungseinheit;
   - Aufnehmen von Trainings-Eingangsdaten durch Messung der von der Trainings-Pflanze reflektierten Strahlung;
   - Trainieren des Klassifikators mit den aufgenommenen Trainings-Eingangsdaten sowie Trainings-Ausgangsdaten, wobei die Trainings-Eingangsdaten zumindest spektrale Informationen, insbesondere ein Absorptionsspektrum oder ein Reflexionsspektrum der Trainings-Pflanze, enthalten und die Trainings-Ausgangsdaten den Trainings-Eingangsdaten zugeordnet sind und Informationen über mindestens eine Eigenschaft der Trainings-Pflanze aufweisen;
- Aufnehmen von Analyse-Eingangsdaten mittels der Sensoreinheit, mit den folgenden Schritten:
   - Beleuchten einer zu analysierenden Pflanze mit mindestens einer unbekannten Eigenschaft unter Verwendung der Beleuchtungseinheit;
   - Aufnehmen von Analyse-Eingangsdaten durch Messung der von der zu analysierenden Pflanze reflektierten Strahlung;
- Bestimmen einer Eigenschaft der zu untersuchenden Pflanze unter Verwendung des zuvor mit den Trainings-Eingangsdaten und Trainings-Ausgangsdaten trainierten Klassifikators und der aufgenommenen Analyse-Eingangsdaten.

Die Beleuchtungseinheit gemäß dem erfindungsgemäßen Verfahren kann insbesondere eine oder mehrere LEDs aufweisen, die in Abhängigkeit der konkreten Ausführungsform sowohl breitbandig, als auch schmalbandig ausgeführt sein können. Die verwendete Sensoreinheit kann insbesondere eine Kamera aufweisen. Auch kann die Sensoreinheit, wie nachfolgend im Zusammenhang mit der erfindungsgemäßen Vorrichtung noch genauer beschrieben wird, ein Spektrometer aufweisen. Die Auswerteeinheit kann insbesondere einen Prozessor und einen Speicher umfassen.

Die spektralen Informationen können insbesondere ein vollständiges Spektrum (zum Beispiel ein Absorptions- oder ein Reflexionsspektrum) beinhalten. Alternativ kann vorgesehen sein, dass die spektralen Informationen lediglich Informationen umfassen, die aus einem aufgenommenen Spektrum extrahiert wurden. Beispielsweise können die spektralen Informationen lediglich Informationen zu einem Absorptionsmaximum oder einem Absorptionsminimum beinhalten, beispielsweise eine Wellenlänge und/oder eine Intensität, die ein Absorptionsmaximum oder ein Absorptionsminimum beschreiben.

Während des Trainierens des Klassifikators "lernt" dieser, wie die Eingangsdaten und die Ausgangsdaten einander zuzuordnen sind. Hierzu dient ein Trainings-Datensatz, der Trainings-Eingangsdaten und Trainings-Ausgangsdaten umfasst. Dieser Trainings-Datensatz wird dadurch gewonnen, dass Trainings-Pflanzen, die mindestens eine bekannte Eigenschaft (z. B. betreffend die Pflanzensorte) aufweisen, verwendet werden und diese vorab mit der Beleuchtungseinheit beleuchtet werden, sodass die anschließend aufgenommenen Trainings-Eingangsdaten (z. B. ein Reflexionsspektrum) und die bekannten Trainings-Ausgangsdaten (z. B. den Wassergehalt einer Pflanze beschreibend) einander zugeordnet werden können. Die Trainings-Ausgangsdaten umfassen dabei Informationen über mindestens eine Eigenschaft der Trainings-Pflanze. Bei dieser Eigenschaft kann es sich insbesondere um den Krankheits- bzw. Gesundheitszustand oder um die Pflanzensorte handeln. Darüber hinaus können die Trainings-Ausgangsdaten Informationen über weitere Eigenschaften der Pflanze, insbesondere über bestimmte Inhaltsstoffe einer Pflanze (beispielsweise der THC-Gehalt von Cannabis) bzw. zu abiotische Faktoren einer Pflanze enthalten. Zu den abiotischen Faktoren gehören zum Beispiel der Nährstoffgehalt in der Pflanze und deren Verteilung innerhalb der Pflanze, Makronährstoffe (insbesondere Stickstoff, Phosphor, Kalium, Calcium, Schwefel, Magnesium, Kohlenstoff, Sauerstoff, Wasserstoff), Mikronährstoffe (insbesondere Vitamine, Mineralstoffe Mengen- und Spurenelemente), und sekundäre Pflanzenstoffe, wie zum Beispiel Eisen, Bor, Chlor, Mangan, Zink, Kupfer, Molybdän und Nickel) und der Wassergehalt der Pflanze.

Ferner können die Ausgangsdaten Informationen zu den Biotischen Stressfaktoren enthalten. Hierzu gehört insbesondere der Befall einer Pflanze von Insekten, Viren und Mehltau.

Sämtliche vorstehend genannten Eigenschaften einer Pflanze, die für das Trainieren des Klassifikators verwendet werden, können anschließend während des Analysevorgangs durch den Klassifikator bestimmt werden.

Bei den vorstehend genannten Eigenschaften einer Pflanze kann es gemäß dem erfindungsgemäßen Verfahren vorgesehen sein, dass die gegenwärtigen Eigenschaften einer Pflanze durch den Klassifikator bestimmt werden. Auch kann vorgesehen sein, dass zukünftige Eigenschaften einer Pflanze durch den Klassifikator bestimmt werden können. Dadurch können Prognosen, insbesondere über den weiteren Verlauf des Wachstumsprozesses einer Pflanze, getroffen werden, die es ermöglichen, den zukünftigen Wachstumsprozess besser vorherzusehen und bei Bedarf durch etwaige Maßnahmen zu beeinflussen. Wenn beispielsweise das erfindungsgemäße Verfahren erkennt, dass die Pflanze einen zu niedrigen Wassergehalt aufweist, kann es daraus schließen, dass sich das zukünftige Wachstum suboptimal entwickeln wird, sodass als Gegenmaßnahme die Bewässerung der Pflanze angepasst werden kann. Auch kann als Reaktion einer negativen Prognose ein Warnsignal ausgegeben und als Gegenmaßnahme beispielsweise die Beleuchtungseinstellungen angepasst werden, sodass die Pflanze optimierte Beleuchtungsbedingungen genießen kann, wodurch der zukünftige Wachstumsprozess positiv beeinflusst werden kann.

Gemäß einer Ausführungsform des erfindungsgemäßen Verfahrens kann vorgesehen sein, dass das erfindungsgemäße Verfahren dazu ausgelegt ist, eine Vorhersage der Entwicklung des Cannabinoidgehalts über die weitere Entwicklungszeit der Pflanze zu treffen. Bei den Cannabinoiden kann es sich insbesondere um THC(A), CBD(A), CBN, CBG, THCV, CBDV handeln. Ferner kann vorgesehen sein, dass das erfindungsgemäße Verfahren dazu ausgelegt ist, den Terpengehalt über die weitere Entwicklungszeit der Pflanze zu bestimmen. Bei den Terpenen kann es sich insbesondere um PNE, MYR, LME, CYE, LNL, HUM, OCM oder TPW handeln.

Das erfindungsgemäße Verfahren strebt an, eine vollautomatisierte Methode bereitzustellen, die eine möglichst präzise Analyse der Pflanzeneigenschaften ermöglicht. Dadurch wird der Wachstumsprozess der Pflanzen besser analysierbar, vorhersagbar und beeinflussbar. Es ist zu erwarten, dass das erfindungsgemäße Verfahren maßgeblich zur Effizienzsteigerung im Pflanzenanbau beitragen kann.

Auch wenn im Zusammenhang mit der Erfindung häufig von einer "Pflanze" die Rede ist, so ist es für den Fachmann ersichtlich, dass damit nicht ausschließlich die gesamte Pflanze gemeint sein muss, sondern dass auch Pflanzenteile, insbesondere ein Pflanzenstil oder Pflanzenblätter, gemeint sein können.

Gemäß einer Ausführungsform der vorliegenden Erfindung kann vorgesehen sein, dass auch mehrere Eigenschaften einer Pflanze bestimmt werden. Beispielsweise kann vorgesehen sein, dass sowohl der Gesundheitszustand, als auch der Wassergehalt einer Pflanze bestimmt wird. Hierzu kann entweder ein einziger Klassifikator eingesetzt werden, der die Bestimmung mehrerer Eigenschaften vornimmt, oder aber auch mehrere Klassifikatoren miteinander kombiniert werden, die sich für die Bestimmung jeweils einer spezifischen Eigenschaft besonders eignen.

Nachdem der Klassifikator mit den Eingangsdaten trainiert wurde, können Pflanzen, die mindestens eine unbekannte Eigenschaft aufweisen, unter Verwendung der Beleuchtungseinheit, der Sensoreinheit und der Auswerteeinheit untersucht werden.

Auch wenn vorstehend das Trainieren des Klassifikators für "eine Pflanze" beschrieben ist, wird bevorzugt eine Vielzahl von Pflanzen verwendet, damit der Trainings-Datensatz möglichst viele Informationen umfasst. Allgemein gilt, dass die Genauigkeit des Klassifikators zunimmt, wenn beim Trainieren möglichst viele Daten vorhanden sind. Beispielsweise kann vorgesehen sein, dass der Schritt des Trainierens des Klassifikators mit einer Vielzahl von Pflanzen, nacheinander wiederholt wird, bis der Klassifikator über eine ausreichende Genauigkeit verfügt. Zur Ermittlung der Erkennungsgenauigkeit bzw. -rate des Klassifikators können beispielsweise Test-Pflanzen eingesetzt werden, deren Eigenschaften bekannt sind und die mit dem trainierten Klassifikators untersucht werden. Da die Eigenschaften der Test-Pflanzen bekannt sind, kann nach der Untersuchung festgestellt werden, wie häufig die Bewertung des Klassifikators korrekt war und wie hoch die Erkennungsrate des Klassifikators ist. Liegt die Erkennungsrate unterhalb einer geforderten Erkennungsrate, beispielsweise unterhalb von 90 % oder 95 %, so kann der Klassifikator mit weiteren Trainings-Pflanzen trainiert werden, bis eine ausreichend hohe Erkennungsrate erreicht wird.

Gemäß einer Ausführungsform des erfindungsgemäßen Verfahrens kann vorgesehen sein, dass zur Erhöhung der Genauigkeit des Verfahrens die Trainings-Eingangsdaten und die Analyse-Eingangsdaten zusätzlich Bildaufnahmen der Trainings-Pflanze und der zu analysierenden Pflanze enthalten. Anders ausgedrückt kann vorgesehen sein, dass die Trainings-Eingangsdaten zusätzlich Bildaufnahmen der Trainings-Pflanze und die Analyse-Eingangsdaten zusätzlich Bildaufnahmen der zu analysierenden Pflanze enthalten. Durch die Bereitstellung zusätzlicher Informationen, die mit den gegenwärtigen und/oder zukünftigen Pflanzeneigenschaften korrelieren, kann die Erkennungsrate des Klassifikators erhöht werden. Darüber hinaus wird es durch die Bereitstellung der Bildaufnahmen ermöglicht, zusätzliche Eigenschaften zu detektieren, die gegebenenfalls bei der ausschließlichen Verwendung der spektralen Informationen nicht zu erkennen sind.

Gemäß einer weiteren Ausführungsform der vorliegenden Offenbarung kann vorgesehen sein, dass die Trainings-Eingangsdaten und die Analyse-Eingangsdaten, unabhängig von spektralen Informationen, Bildaufnahmen der Trainings-Pflanze und der zu analysierenden Pflanze enthalten. Anders ausgedrückt ist es gemäß dieser Ausführungsform nicht zwingend notwendig, dass die Eingangsdate spektrale Informationen enthalten.

Gemäß einer weiteren Ausführungsform des erfindungsgemäßen Verfahrens kann vorgesehen sein, dass das Trainieren des Klassifikators zusätzlich die folgenden Schritte aufweist:
- Bereitstellen von Bildaufnahmen als Trainings-Eingangsdaten, die für das Trainieren des Klassifikators verwendet werden sollen und von Trainings-Ausgangsdaten, die den bereitgestellten Bildaufnahmen zugeordnet sind;
- Rotieren der bereitgestellten Bildaufnahmen;
- Zuordnen der Trainings-Ausgangsdaten, die den ursprünglich bereitgestellten Bildaufnahmen zugeordnet sind, zu den rotierten Bildaufnahmen;
- Zusammenfügen der ursprünglich bereitgestellten Bildaufnahmen und der rotierten Bildaufnahmen sowie der den Bildaufnahmen zugeordneten Trainings-Ausgangsdaten zu einem erweiterten Trainings-Datensatz; und
- Trainieren des Klassifikators unter Verwendung des erweiterten Trainings-Datensatzes.

Wie bereits vorstehend erläutert, ist die Erkennungsrate des Klassifikators in der Regel höher, wenn besonders umfangreiche Trainings-Daten vorliegen. Um die Genauigkeit des Klassifikators zu erhöhen, kann der Trainings-Datensatz in bevorzugter Weise "künstlich" erweitert werden. Liegen beispielsweise im Trainings-Datensatz lediglich zehn Bildaufnahmen von Trainings-Pflanzen vor, können zu jedem der Bildaufnahmen durch die vorstehende Maßnahme beispielsweise 100 zusätzliche Bildaufnahmen generiert werden, bei denen die ursprünglich bereitgestellte Bildaufnahme jeweils schrittweise rotiert ist. Bei der Rotation der Bilder kann insbesondere vorgesehen sein, dass die ursprünglich bereitgestellten Bildaufnahmen um eine Rotationsachse gedreht werden, die senkrecht zur Bildfläche verläuft. Auf diese Weise kann die Erweiterung des Datensatzes ohne jegliche Änderung des Versuchsstandes vorgenommen werden. In diesem Zusammenhang kann also von einer "softwarebasierten Erweiterung" des Trainings-Datensatzes gesprochen werden.

Alternativ kann es vorgesehen sein, dass bereits während der Aufnahme der Trainings-Eingangsdaten bzw. der Bilder entweder die Pflanze oder aber auch die Kamera gedreht bzw. geschwenkt werden, um Bildaufnahmen in unterschiedlichen Perspektiven zu erhalten. Auf diese Weise kann ebenfalls der Trainings-Datensatz erweitert werden, ohne zusätzliche Trainings-Pflanzen bereitzustellen zu müssen. Hierbei kann zusätzlich vorgesehen sein, dass entweder die für die Aufnahmen verwendete Kamera oder die Trainings-Pflanze automatisiert geschwenkt bzw. rotiert werden, um auf diese Weise einen erweiterten Trainings-Datensatz automatisch zu generieren. Insbesondere kann dabei vorgesehen sein, dass die Kamera entlang einer Kreisbahn verfahrbar und zudem schwenkbar angeordnet ist und dass in bestimmten Zeitabständen Bildaufnahmen der Trainings-Pflanze erfasst werden. In diesem Zusammenhang kann also von einer "hardwarebasierten Erweiterung" des Trainings-Datensatzes gesprochen werden.

Bei der Erweiterung des Trainings-Datensatzes kann es beispielsweise vorgesehen sein, dass zunächst ein erweiterter Datensatz erstellt und (beispielsweise auf einer Festplatte) abgespeichert wird, bevor der Trainingsprozess mit dem erweiterten Datensatz beginnt. Alternativ kann aber auch vorgesehen sein, dass der erweiterte Datensatz nicht (permanent) abgespeichert wird, sondern die generierten erweiterten Daten unmittelbar dazu verwendet werden, den Klassifikator zu trainieren. Auf diese Weise kann der Klassifikator direkt nach der Erzeugung eines neuen Bildes mit den neuen Daten trainiert werden, ohne diese Daten abzuspeichern. Dies kann dann von Vorteil sein, wenn bei der Erweiterung des Trainings-Datensatzes eine besonders große Datenmenge erwartet wird, zeitgleich jedoch der zur Verfügung stehende Speicherplatz limitiert ist.

Der erweiterte Trainings-Datensatz umfasst also einerseits die ursprünglich bereitgestellten Bildaufnahmen (Trainings-Eingangsdaten) und die diesen Bildaufnahmen zugeordneten Trainings-Ausgangsdaten sowie andererseits die "manipulierten" Bildaufnahmen, denen die gleichen Trainings-Ausgangsdaten zugeordnet sind, wie den ursprünglich bereitgestellten Bildaufnahmen.

Gemäß einer weiteren Ausführungsform des erfindungsgemäßen Verfahrens, kann vorgesehen sein, dass der Trainings-Datensatz dadurch erweitert wird, dass die ursprünglich bereitgestellten Bildaufnahmen gespiegelt werden. Dabei kann vorgesehen sein, dass die Bildaufnahmen um eine horizontale oder eine vertikale Achse gespiegelt werden.

Ferner kann vorgesehen sein, dass ein wesentliches bzw. charakteristisches Element der Bildaufnahmen, beispielsweise ein Pflanzenblatt, innerhalb des Bildes verschoben wird. Beispielsweise kann sich bei einer ursprünglich bereitgestellten Bildaufnahme ein aufgenommenes Blatt einer Pflanze in einem unteren, linken Bereich des Bildes befinden. Um den Trainings-Datensatz zu erweitern kann daher das Pflanzenblatt innerhalb des Bildes in verschiedene Positionen, beispielsweise in die Mitte des Bildes oder in den oberen, rechten Bereich des Bildes, verschoben werden.

Gemäß einer weiteren vorteilhaften Ausführungsform der Erfindung kann vorgesehen sein, dass zur Erweiterung des Trainings-Datensatzes ein wesentliches bzw. charakteristisches Element der Bildaufnahmen skaliert wird. Hierzu kann einerseits vorgesehen sein, dass der Abstand zwischen einer Kamera und dem aufzunehmenden Objekt (beispielsweise die Pflanze oder ein Pflanzenblatt) während der Aufnahme der Bilder variiert. Alternativ kann auch vorgesehen sein, dass der Abstand zwischen der Kamera und dem aufzunehmenden Objekt konstant bleibt, wobei der Skalierungseffekt durch eine Zoom-Funktion der Kamera erfolgt. In den beiden vorstehend beschriebenen Fällen kann auch von einer "hardwarebasierten Skalierung" gesprochen werden. Ferner kann vorgesehen sein, dass die bereitgestellten Bildaufnahmen ein charakteristisches Element aufweisen, welches nach der Aufnahme der Bilder digital bearbeitet wird. In diesem Zusammenhang kann von einer "softwarebasierten Skalierung" gesprochen werden.

Gemäß einer weiteren alternativen Ausführungsform des erfindungsgemäßen Verfahrens kann es vorgesehen sein, dass zur Erweiterung des Trainings-Datensatzes die Helligkeit des Bildes variiert wird. Hierzu kann einerseits bereits während der Aufnahme der Bilder die Beleuchtungseinheit derart angepasst werden, dass die zu analysierenden Pflanze unterschiedlich stark beleuchtet wird. Es können also diverse Aufnahmen bei verschiedenen Belichtungsbedingungen durch die Sensoreinheit, insbesondere durch eine Kamera, erfasst werden. Alternativ hierzu kann es vorgesehen sein, dass die Bilder unter denselben Bedingungen beleuchtet werden und anschließend die Helligkeit digital nachbearbeitet wird. Die digitale Nachbearbeitung hat den Vorteil, dass ein umfangreicher Trainings-Datensatz mit relativ wenig Aufwand zusammengestellt werden kann.

Auch kann vorgesehen sein, dass zur Erweiterung des Trainings-Datensatzes die Bildaufnahmen mit einem Rauschsignal überlagert werden. Bei dem Rauschsignal kann es sich beispielsweise um weißes Rauschen handeln.

Gemäß einer besonders bevorzugten Ausführungsform des erfindungsgemäßen Verfahrens kann es vorgesehen sein, dass zur Erhöhung der Genauigkeit des Verfahrens die Trainings-Eingangsdaten und die Analyse-Eingangsdaten zusätzlich genetische Informationen von Pflanzen enthalten. Dadurch kann die Zuverlässigkeit und Präzision des Klassifikators erhöht werden, indem während der Trainingsphase zusätzliche Informationen zur DNA einer Pflanze bereitgestellt werden. Eine Berücksichtigung der genetischen Informationen kann die Präzision des Klassifikators besonders erhöhen, da viele Eigenschaften von Pflanzen, unter anderem die Resistenz gegenüber Bakterien, Viren und anderen Krankheitserregern und die Empfindlichkeit gegenüber Temperatur- oder Feuchtigkeitsänderungen wesentlich von der Veranlagung der Pflanze abhängen. Die Berücksichtigung genetischer Informationen ist insbesondere bei Cannabis vorteilhaft. Ein Grund hierfür liegt darin, dass Cannabis in der Regel in Gewächshäusern und Klimakammern angebaut wird, wobei in der Regel eine Vielzahl von Pflanzen vorhanden ist, die genetisch identisch sind. Wenn die genetischen Informationen der angebauten Pflanze bekannt ist und der Trainings-Datensatz, welcher zum Trainieren des Klassifikators eingesetzt wurde, ebenfalls Pflanzen mit derselben DNA umfasste, so führt die Berücksichtigung genetischer Informationen zu einer signifikanten Verbesserung des erfindungsgemäßen Verfahrens. In der Regel ist es im vorstehend beschriebenen Szenario mit genetisch identischen Pflanzen nicht erforderlich, die genetischen Informationen der zu analysierenden Pflanze jedes Mal neu zu ermitteln. Vielmehr kann vorgesehen sein, dass aus einer vorgegebenen Liste der entsprechende Typ der Pflanze und entsprechend der Auswahl die genetischen Informationen der zu analysierenden Pflanze als Teil der Analyse-Eingangsdaten eingelesen und an den Klassifikator weitergeleitet werden. Bei den genetischen Informationen kann beispielsweise die gesamte DNA-Information verwendet werden, oder aber auch einzelne DNA-Sequenzen, die in Verbindung mit einer bestimmten Eigenschaft stehen. Beispielsweise können bestimmte DNA-Sequenzen mit einer hohen Resistenz gegenüber bestimmten Krankheitserregern korrelieren.

Ferner kann nach einer Ausführungsform des erfindungsgemäßen Verfahrens vorgesehen sein, dass der Klassifikator auf einem künstlichen neuronalen Netz, insbesondere auf einem Convolutional Neural Network (CNN), basiert. Erste Untersuchungen haben bereits gezeigt, dass neuronale Netze, insbesondere sogenannte Convolutional Neural Networks (im Deutschen auch als "faltendes neuronales Netz" bezeichnet) im Zusammenhang mit dem erfindungsgemäßen Verfahren zu hohen Erkennungsraten führen und daher besonders geeignet sind. CNNs sind insbesondere im Zusammenhang mit multidimensionalen Daten prädestiniert und eignen sich daher besonders gut in Kombination mit dem erfindungsgemäßen Verfahren.

Auch kann vorgesehen sein, dass der Klassifikator auf einem der nachfolgend genannten Verfahren basiert: Rekurrentes Neuronales Netz (RNN), Long shortterm memory (auch als LSTM oder im deutschen als "langes Kurzzeitgedächtnis" bezeichnet), Supervised Learning (im Deutschen auch als "überwachtes Lernen" bezeichnet), Unsupervised Learning (entsprechend im Deutschen als "unüberwachtes Lernen" bezeichnet) und Machine Learning (im Deutschen: Maschinelles Lernen). Ferner kann vorgesehen sein, dass der Klassifikator auf einer Kombination mehrerer der vorstehend genannten Verfahren basiert.

Gemäß einer Ausführungsform des erfindungsgemäßen Verfahrens kann vorgesehen sein, dass zur Erhöhung der Genauigkeit des Verfahrens beim Trainieren des Klassifikators die Trainings-Eingangsdaten und die Analyse-Eingangsdaten Informationen über die zeitliche Änderung der aufgenommenen Eingangsdaten aufweisen. Dies kann die Genauigkeit der Analyse weiter erhöhen, wie nachfolgend erläutert wird. In bestimmten Situationen kann es für den Klassifikator schwierig sein, die Eigenschaft einer Pflanze, beispielsweise den Gesundheits- und Krankheitszustand einer Pflanze, zu erkennen. Dies liegt daran, dass die aktuell gemessenen Werte an und für sich in einem akzeptablen und tolerierten Bereich liegen können, während sie jedoch eine abrupte zeitliche Änderung zeigen. Beispielsweise kann es sein, dass ein gemessener Absorptionspeak (bzw. die Wellenlänge und die Intensität eines gemessenen Absorptionspeaks) in einem Bereich liegt, der üblicherweise einer gesunden Pflanze zuzuordnen ist. Dennoch kann die zeitliche Änderung des gemessenen Absorptionspeaks einen Anhaltspunkt dafür liefern, dass die Pflanze bereits von einem Krankheitserreger befallen ist. Daher kann die Berücksichtigung der Informationen über die zeitliche Änderung der aufgenommenen Messdaten zu einer signifikanten Verbesserung des erfindungsgemäßen Verfahrens führen. Insbesondere können Krankheiten und weitere Probleme beim Wachstum einer Pflanze erkannt werden, die sonst nur schwer zu erkennen wären. Dabei kann beispielsweise vorgesehen sein, dass die Trainings-Eingangsdaten Daten umfassen, welche die zeitliche Differenz der aufgenommenen Messdaten wiedergeben. Beispielsweise kann hierzu die Differenz aus einem Absorptionsspektrum, das zum Zeitpunkt t aufgenommen wurde, und einem Absorptionsspektrum, das zum Zeitpunkt t - n aufgenommen wurde, berechnet werden und der Klassifikator mit Daten trainiert werden, die der Differenz beider Spektren entsprechen. Ferner kann vorgesehen sein, dass nicht die gesamten Spektren voneinander subtrahiert werden, sondern lediglich ein Merkmal bzw. mehrere Merkmale, die aus den aufgenommenen Spektren extrahiert wurden. So kann beispielsweise die Lage (bzw. die Wellenlänge) eines Absorptionspeaks zum Zeitpunkt t mit der Lage (bzw. der Wellenlänge) eines Absorptionspeaks zum Zeitpunkt t - n verglichen und die Differenz hieraus berechnet werden. Dadurch kann also ein zeitlicher "Sprung" eines Absorptionspeaks detektiert werden, der beispielsweise im Zusammenhang mit der Krankheit einer Pflanze stehen kann.

Gemäß einer weiteren bevorzugten Ausführungsform des erfindungsgemäßen Verfahrens kann vorgesehen sein, dass zur Verbesserung des Klassifikators Trainingsdaten eingesetzt werden, die Umweltdaten umfassen. Zu den Umweltdaten gehören insbesondere die Luft- und/oder Bodentemperatur, die Luftfeuchtigkeit, der CO2-Gehalt der Luft, der atmosphärische Druck, die Nährstoffversorgung der Pflanze (durch Wasser, Boden und/oder Nährmedium), die Lichtverhältnisse (sowohl in einem Gewächshaus, als auch im Freien) und Wetterinformationen (beispielsweise sonnig, bewölkt, regnerisch und/oder windig). Bei den Umweltdaten können insbesondere die gegenwärtigen Daten, die Daten aus der Vergangenheit und/oder die zukünftigen Daten berücksichtigt werden, um eine möglichst genaue Analyse der gegenwärtigen oder aber auch der zukünftigen Eigenschaften der Pflanze zu gewährleisten. Bei den zukünftigen Daten können insbesondere Informationen über eine Wettervorhersage mit einfließen.

Des Weiteren wird zur Lösung der vorstehend genannten Aufgabe eine Vorrichtung zur Analyse einer Pflanze, insbesondere einer Hanfpflanze, vorgeschlagen, die Folgendes umfasst:
- eine Beleuchtungseinheit zur Beleuchtung der zu analysierenden Pflanze;
- eine Sensoreinheit zur Aufnahme von Analyse-Eingangsdaten, wobei die Analyse-Eingangsdaten zumindest spektrale Informationen, insbesondere ein Absorptionsspektrum oder ein Reflexionsspektrum der Trainings-Pflanze, enthalten; und
- eine Auswerteeinheit zur Auswertung der aufgenommenen Analyse-Eingangsdaten und zum Bestimmen mindestens einer Eigenschaft der zu analysierenden Pflanze; wobei
- die Auswerteeinheit dazu ausgelegt ist, die mindestens eine Eigenschaft der Pflanze unter Verwendung eines datenbasierten Klassifikators sowie der zuvor aufgenommenen Analyse-Eingangsdaten zu bestimmen.

Die Beleuchtungseinheit kann insbesondere eine oder mehrere LEDs umfassen. LEDs haben insbesondere den Vorteil, dass sie leicht anzusteuern, kostengünstig verfügbar und in einer Vielzahl unterschiedlicher Eigenschaften (insbesondere unterschiedlicher Emissionsspektren) verfügbar sind. Beispielsweise kann vorgesehen sein, dass die Beleuchtungseinheit eine Vielzahl von LEDs mit jeweils unterschiedlichen Emissionsspektren aufweist. Dabei können die einzelnen LEDs schmalbandige Emissionsspektren aufweisen. Die Emissionsspektren der einzelnen LEDs können sich insgesamt über den UV-Bereich, den sichtbaren und den infraroten Bereich erstrecken. Beispielsweise können insgesamt 12 LEDs mit jeweils schmalbandingen Emissionsspektren eingesetzt werden, deren Emissionsspektrum insgesamt einen Wellenlängenbereich von 290 bis 950 nm umfasst. Alternativ kann vorgesehen sein, dass die Beleuchtungseinheit eine breitbandige Lichtquelle, insbesondere eine breitbandige LED aufweist.

Die Sensoreinheit ist dazu ausgelegt, spektrale Informationen der zu analysierenden Pflanze aufzunehmen. Beispielsweise kann hierfür ein Spektrometer vorgesehen sein. Die Verwendung eines "klassischen" Spektrometers ist jedoch nicht zwingend erforderlich. Während ein klassisches Spektrometer eine unmittelbare Messung eines Spektrums (insbesondere eines Absorptions- oder eines Reflexionsspektrums) ermöglicht, gibt es alternative Möglichkeiten, spektrale Informationen auch ohne ein klassisches Spektrometer (mittelbar) zu erhalten.

Gemäß einer Ausführungsform der erfindungsgemäßen Analysevorrichtung kann beispielsweise vorgesehen sein, dass die Sensoreinheit lediglich eine Kamera aufweist. Die Beleuchtungseinheit kann hierzu beispielsweise 10 LEDs aufweisen, die jeweils ein schmalbandiges Emissionsspektrum mit jeweils unterschiedlichen zentralen Wellenlängen aufweisen. Die zentralen Wellenlängen der LEDs können beispielsweise jeweils ca. 100 nm auseinanderliegen, sodass das gesamte Emissionsspektrum sämtlicher LEDs ca. 1000 nm umfassen kann. Zur Aufnahme der spektralen Informationen kann gemäß einer Ausführungsform der erfindungsgemäßen Vorrichtung vorgesehen sein, dass die zu analysierenden Pflanze mittels einer ersten LED beleuchtet wird und das von der Pflanze reflektierte Licht mittels einer Kamera aufgenommen wird. Anschließend kann die zu analysierenden Pflanze mittels einer zweiten LED (mit einem von der ersten LED unterschiedlichen Emissionsspektrum) beleuchtet werden und das von der Pflanze emittierte Licht erneut aufgenommen werden. Dieser Vorgang kann wiederholt werden, bis für sämtliche der zehn LEDs eine Kameraaufnahme vorliegt. Anschließend können die Daten der Kamera ausgewertet werden. Insbesondere kann es vorgesehen sein, dass die Kamera zu jedem Pixel ein RGB-Signal aufnimmt. Daher kann die Lichtintensität der einzelnen RGB-Subpixel verwendet werden, um Informationen zu dem Reflexionsspektrum zu erhalten, der eine Abhängigkeit von der verwendeten LED aufweist. Auf diese Weise können spektrale Informationen aufgenommen werden, ohne ein "klassisches" Spektrometer zu verwenden. Die zu analysierende Pflanze kann also auf diese Weise sequenziell schmalbandig beleuchtet und das gemessene Spektrum breitbandig aufgenommen werden. Dieser Aufbau ermöglicht eine sehr kostengünstige und schnelle Analyse der Pflanzen. Anders ausgedrückt wird auf diese Weise also ein Spektrum "abgetastet".
Die Auswerteeinheit gemäß der erfindungsgemäßen Analysevorrichtung kann insbesondere einen Speicher zum Speichern der Trainings- und/oder AnalyseDaten sowie einen Prozessor zur Durchführung des vorstehend beschriebenen erfindungsgemäßen Verfahrens aufweisen.

Bei dem im Zusammenhang mit der erfindungsgemäßen Analysevorrichtung verwendeten Klassifikator kann insbesondere einer der vorstehend im Zusammenhang mit dem erfindungsgemäßen Verfahren beschriebenen Klassifikatoren bzw. Klassifikationsverfahren eingesetzt werden.

Gemäß einer bevorzugten Ausführungsform der erfindungsgemäßen Vorrichtung kann vorgesehen sein, dass die Sensoreinheit ein Spektrometer aufweist. Durch die Verwendung eines Spektrometers wird die Aufnahme hochauflösender spektraler Informationen ermöglicht. Durch die Verwendung hochauflösender spektraler Informationen beim Trainieren des Klassifikators wird es ermöglicht, die Präzisierung und die Zuverlässigkeit des Klassifikators zu erhöhen.

Gemäß einer besonders bevorzugten Ausführungsform der erfindungsmäßen Vorrichtung kann vorgesehen sein, dass die Sensoreinheit eine Kamera, insbesondere eine CMOS-Kamera, umfassend eine Sensorfläche aufweist. Insbesondere kann vorgesehen sein, dass die verwendete Kamera eine spezielle Antireflexionsschicht aufweist, um ein möglichst starkes Signal aufzunehmen und ein verbessertes Signal-zu-Stör-Verhältnis (SNR - signal to noise ratio) zu ermöglichen und dadurch die Präzision und Zuverlässigkeit des Klassifikators zu erhöhen.

Ferner kann vorgesehen sein, dass die Kamera der erfindungsgemäßen Analysevorrichtung als 3D-Kamera, insbesondere als Stereokamera, ausgeführt ist. Die Verwendung einer 3D-Kamera ermöglicht es insbesondere, Informationen über das Volumen einer Pflanze zu ermitteln. Hierzu kann beispielsweise ein zu untersuchendes Pflanzenblatt auf einer planen Unterlage abgelegt und die Kontur des Blattes durch die 3D-Kamera aufgenommen werden. Aus den aufgenommenen Daten kann dann unmittelbar das Volumens des Blattes berechnet werden. In diesem Zusammenhang kann beispielsweise vorgesehen sein, dass die erfindungsgemäße Vorrichtung dazu verwendet wird, die THC-Konzentration von zu analysierenden Cannabis-Blättern zu ermitteln. Durch die Verwendung der 3D-Kamera und die Möglichkeit der Volumenbestimmung wird es dann ermöglicht, nicht nur die THC-Konzentration zu ermitteln, sondern auch direkt die absolute Menge an THC. Diese kann also durch einfache Multiplikation der THC-Konzentration und dem Volumen des Pflanzenblattes ermittelt werden. So kann auf einfache Art und Weise bestimmt werden, wie viel THC ein Cannabis-Blatt oder eine ganze Pflanzencharge aufweist.

Ein weiterer Vorteil des Einsatzes einer 3D-Kamera ist darin zu sehen, dass diese es ermöglicht, die Anordnung der Pflanze und der Kamera (relativ zueinander) zu ermitteln. Dadurch kann der Abstrahlwinkel des von der Pflanze reflektierten Lichtes berechnet werden, wobei dieser Abstrahlwinkel in die Trainings-Eingangsdaten sowie den Analyse-Eingangsdaten einfließen kann.

Gemäß einer bevorzugten Ausführungsform der erfindungsgemäßen Vorrichtung kann vorgesehen sein, dass mehrere 3D-Kameras eingesetzt werden, welche die zu untersuchende Pflanze aus unterschiedlichen Richtungen aufnehmen. Dadurch wird es ermöglicht, das Volumen einer Pflanze oder eines Blattes möglichst präzise zu ermitteln. Entsprechend kann auf diese Weise auch die Bestimmung des absoluten THC-Gehaltes besonders präzise erfolgen.

Gemäß einer Ausführungsform der erfindungsgemäßen Vorrichtung kann vorgesehen sein, dass eine Stereokamera eingesetzt wird. Alternativ kann vorgesehen sein, dass ein auf structured light (auf Deutsch "strukturiertes Licht") basierendes Verfahren zur Ermittlung der dreidimensionalen Form bzw. zur Bestimmung des Volumens der zu untersuchenden Pflanze eingesetzt wird.

Ferner kann vorgesehen sein, dass die Beleuchtungseinheit der erfindungsgemäßen Vorrichtung mindestens zwei Beleuchtungselemente, insbesondere mindestens zwei LEDs, aufweist. Diese können insbesondere unterschiedliche Emissionsspektren zeigen. Gemäß einer bevorzugten Ausführungsform kann vorgesehen sein, dass eine Vielzahl, beispielsweise zehn oder 20 LEDs mit unterschiedlichen Emissionsspektren eingesetzt werden. Die Verwendung einer Mehrzahl von LEDs unterschiedlicher Emissionswellenlänge ist insbesondere vorteilhaft, wenn die erfindungsgemäße Vorrichtung kein klassisches Spektrometer verwendet, sondern die spektralen Informationen einer Pflanze (wie vorstehend erläutert) durch die einzelnen LEDs "abgetastet" werden.

Darüber hinaus kann vorgesehen sein, dass die Beleuchtungselemente der erfindungsgemäßen Vorrichtung auf einer Kreisbahn angeordnet sind, welche die Sensorfläche der Kamera umgibt. Dadurch kann eine besonders homogene Ausleuchtung der zu untersuchenden Pflanze gewährleistet werden. Ein weiterer Vorteil der Anordnung der Beleuchtungselemente auf einer die Sensorfläche der Kammer umgebenden Kreisbahn ist, dass durch die homogene Beleuchtung der zu analysierenden Pflanze etwaige Unregelmäßigkeiten bei der Aufnahme der Trainings- und Analyse-Eingangsdaten reduziert werden. Dies hat positive Auswirkungen auf die Erkennungsrate des Klassifikators.

Auch kann vorgesehen sein, dass die Sensoreinheit und/oder die Beleuchtungseinheit der erfindungsgemäßen Vorrichtung ein Kühlelement aufweisen. Durch die Verwendung eines Kühlelements kann insbesondere das Rauschen der Sensoreinheit und/oder der Beleuchtungseinheit reduziert werden. Dadurch kann die Präzision und Zuverlässigkeit des Klassifikators verbessert werden. Beispielsweise kann vorgesehen sein, dass das Kühlelement als Peltier-Element ausgeführt ist oder ein Peltier-Element aufweist.

Gemäß einer Ausführungsform der erfindungsgemäßen Vorrichtung kann vorgesehen sein, dass die Kühleinheit eine Regelungseinheit aufweist, welche die Sensoreinheit und/oder Beleuchtungseinheit oder zumindest ein Element der Sensoreinheit und/oder der Kühleinheit auf eine vorgebbare Temperatur kühlt.

Gemäß einer weiteren erfindungsgemäßen Ausführungsform der Analysevorrichtung kann vorgesehen sein, dass die Sensoreinheit zusätzlich einen Temperatursensor und/oder einen Feuchtigkeitssensor aufweist. Auch kann vorgesehen sein, dass die Sensoreinheit zusätzlich Daten betreffend die Wettervorhersage erfasst.

Gemäß einer Ausführungsform der erfindungsgemäßen Analysevorrichtung kann zudem vorgesehen sein, dass der Temperatursensor eine Wärmebildkamera aufweist.

Ferner kann vorgesehen sein, dass die Sensoreinheit der erfindungsgemäßen Vorrichtung eine mit einer UV-zu-VIS Umwandlungsbeschichtung versehene Sensorfläche aufweist. Dabei kann es sich bei der Sensorfläche insbesondere um die Sensorfläche einer Kamera handeln. Durch die Verwendung einer UV-zu-VIS Umwandlungsbeschichtung kann die Empfindlichkeit der Sensoreinheit, insbesondere der Kamera, im UV-Bereich erhöht werden. Dies ist insbesondere deshalb vorteilhaft, weil kommerziell verfügbare CCD- und CMOS-Kameras im UV-Bereich üblicherweise eine sehr geringe Lichtausbeute zeigen. Daher kann gemäß der bevorzugten Ausführungsform der erfindungsgemäßen Vorrichtung vorgesehen sein, dass die Sensorfläche mit einer dünnen Schicht eines Materials versehen ist, welches UV-Licht absorbiert und anschließend sichtbares Licht emittiert. Insbesondere kann vorgesehen sein, dass die Sensorfläche mit einer dünnen Schicht 1-Naphthalenecarboxaldehyde, 2-hydroxy-, [(2-hydroxy-1-naphthalenyl)methylene]hydrazone(9CI) versehen ist. Insbesondere kann diese Schicht mittels physikalischer Gasphasenabscheidung (englisch: physical vapour deposition, oder kurz: PVD) auf die Sensorfläche aufgebracht werden. Durch die Verwendung der UV-zu-VIS Beschichtung kann die Erkennungsrate des Klassifikators deutlich erhöht werden.

Vorstehend wurde das erfindungsgemäße Verfahren der Vollständigkeit halber im Zusammenhang mit dem Trainingsprozess und dem Analyseprozess beschrieben. Es wird als selbstverständlich angesehen, dass der Vorgang des Trainierens nicht zwingend beim Benutzer erfolgen muss, sondern dass ein Anbieter des erfindungsgemäßen Verfahrens sowie der erfindungsgemäßen Vorrichtung den Trainingsprozess vollständig übernehmen kann, sodass der Benutzer, ohne vorheriges Training, das erfindungsgemäße Verfahren bzw. die erfindungsgemäße Vorrichtung verwenden kann. Dementsprechend wird zur Lösung der vorstehend genannten Aufgabe gemäß einer weiteren Ausführungsform der Erfindung ein Verfahren zur Analyse einer Pflanze, insbesondere zur Analyse von Cannabis, unter Verwendung einer Beleuchtungseinheit, einer Sensoreinheit und einer Auswerteeinheit vorgeschlagen, wobei die Auswerteeinheit einen datenbasierten Klassifikator enthält und das Verfahren die nachfolgenden Schritte aufweist:
- Beleuchten einer Analyse-Pflanze mit mindestens einer unbekannten Eigenschaft unter Verwendung der Beleuchtungseinheit;
- Aufnehmen von Analyse-Eingangsdaten durch Messung der von der Analyse-Pflanze reflektierten Strahlung unter Verwendung einer Sensoreinheit; wobei
- die Analyse-Eingangsdaten zumindest spektrale Informationen, insbesondere ein Absorptionsspektrum oder ein Reflexionsspektrum der Analyse-Pflanze, enthalten;
- Bestimmen einer Eigenschaft der zu analysierenden Pflanze unter Verwendung eines zuvor mit Trainings-Eingangsdaten und Trainings-Ausgangsdaten trainierten Klassifikators und der aufgenommenen Analyse-Eingangsdaten, wobei die Trainings-Ausgangsdaten den Trainings-Eingangsdaten zugeordnet sind und Informationen über mindestens eine Eigenschaft der Trainings-Pflanze aufweisen.

Zudem wird es als selbstverständlich angesehen, dass das vorstehend beschriebene Verfahren zur Analyse einer Pflanze, welches keinen Trainingsprozess umfasst, auch mit sämtlichen Ausführungsbeispielen, die vorstehend in Kombination mit dem Trainingsprozess beschrieben wurden, kompatibel und kombinierbar ist.

Ferner wird es als selbstverständlich angesehen, dass sämtliche vorstehend erläuterten Schritte des erfindungsgemäßen Verfahrens mit der erfindungsgemäßen Vorrichtung kombinierbar sind und andersherum genauso sämtliche Ausführungsformen der erfindungsgemäßen Vorrichtung mit dem erfindungsgemäßen Verfahren kombinierbar sind.

Die Erfindung wird nachfolgend anhand von Ausführungsbeispielen und unter Bezugnahme auf die Zeichnungen näher erläutert. Im Einzelnen zeigen dabei:
- Fig. 1: eine Prinzipdarstellung eines ersten Ausführungsbeispiels der Erfindung,
- Fig. 2: eine Prinzipdarstellung eines zweiten Ausführungsbeispiels der Erfindung,
- Fig. 3: eine Prinzipdarstellung eines dritten Ausführungsbeispiels der Erfindung,
- Fig. 4: eine Draufsicht auf eine erste Ausführungsform der Beleuchtungseinheit der erfindungsgemäßen Analysevorrichtung,
- Fig. 5: eine Draufsicht auf eine zweite Ausführungsform der Beleuchtungseinheit der erfindungsgemäßen Analysevorrichtung, wobei die Sensoreinheit in der Beleuchtungseinheit integriert ist,
- Fig. 6: ein Flussdiagramm zur Darstellung des erfindungsgemäßen Verfahrens,
- Fig. 7: ein weiteres Flussdiagramm zur Darstellung der einzelnen Schritte des Trainierens des Klassifikators,
- Fig. 8: eine Prinzipdarstellung eines aufgenommenen Reflexionsspektrums,
- Fig. 9: eine Prinzipdarstellung einer ersten Ausführungsform des Klassifikators und
- Fig. 10: eine Prinzipdarstellung einer zweiten Ausführungsform des Klassifikators.

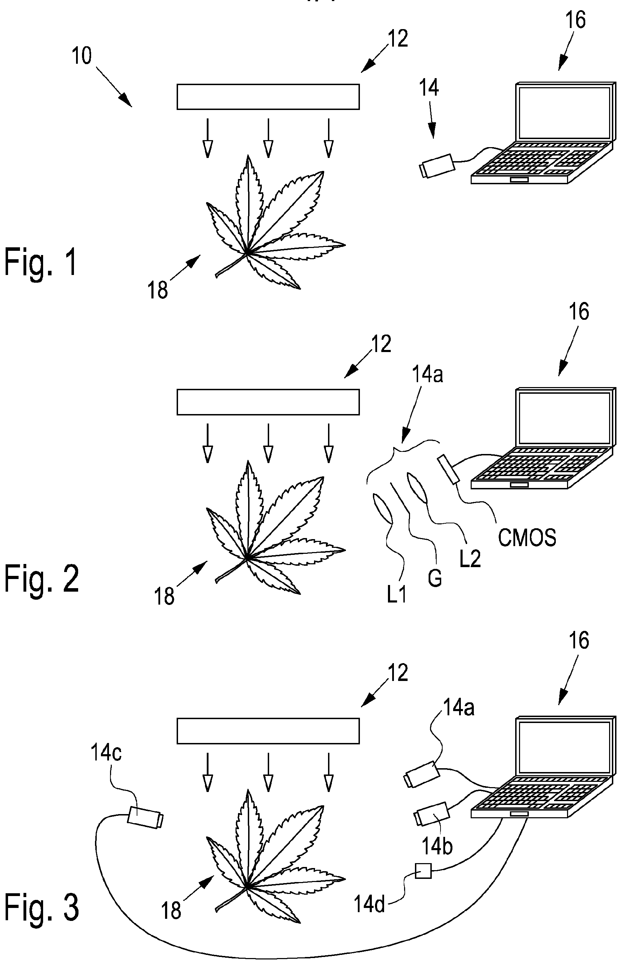

In der Fig. 1 ist ein erstes Ausführungsbeispiel der erfindungsgemäßen Analysevorrichtung 10 dargestellt. Die Analysevorrichtung 10 umfasst eine Beleuchtungseinheit 12, eine Sensoreinheit 14 sowie eine Auswerteeinheit 16. Die Sensoreinheit 14 kann beispielsweise als Kamera oder als Spektrometer ausgeführt sein. Die Auswerteeinheit 16 ist in dem dargestellten Ausführungsbeispiel als ein tragbarer Computer ausgeführt. Die Analysevorrichtung 10 ist dazu ausgelegt, eine Pflanze (bzw. ein Pflanzenblatt) 18 zu analysieren. Hierzu wird die Pflanze 18 durch die Beleuchtungseinheit 12 bestrahlt. Die von der Pflanze 18 reflektierte Strahlung wird anschließend durch die Sensoreinheit 14 aufgenommen. Die von der Sensoreinheit 14 aufgenommenen Daten werden durch die Auswerteeinheit 16 unter Verwendung eines Klassifikators ausgewertet, welcher in dieser Fig. nicht gezeigt ist. Die Sensoreinheit 14 ist insbesondere dazu ausgelegt, spektrale Informationen zu erfassen. Diese können entweder unmittelbar, beispielsweise durch ein Spektrometer aufgenommen werden, oder aber auch mittelbar, z.B. durch Beleuchten der Pflanze 18 mit LEDs unterschiedlicher Emissionsspektren und anschließender Aufnahme der Intensitäten durch eine RGB-Kamera. Dieses "Abtasten" des Spektrums kann insbesondere auf der bereits vorstehend beschriebenen Art erfolgen. Insofern ist es nicht zwingt erforderlich, dass die Sensoreinheit 14 ein Spektrometer aufweist.

In der Fig. 2 ist ein zweites Ausführungsbeispiel der erfindungsgemäßen Analysevorrichtung 10 dargestellt. Bei diesem Ausführungsbeispiel ist die Sensoreinheit 14 als Spektrometer 14a ausgeführt. Das Spektrometer 14a umfasst eine erste Linse L1, ein Beugungsgitter G, eine zweite Linse L2 sowie eine CMOS-Kamera. Die erste Linse L1 dient dazu, dass von der Pflanze 18 reflektierte Licht zu kollimieren. Das kollimierte Licht durchläuft anschließend das Beugungsgitter G. Das Beugungsgitter G zerlegt das einfallende Licht in seine spektralen Anteile und lenkt die Anteile unterschiedlicher Wellenlängen auf verschiedene Bereiche der CMOS-Kamera. Somit erfolgt eine räumliche "Auffächerung" des Lichts auf die Sensorfläche der CMOS-Kamera. Durch die anschließende Auswertung des von der CMOS-Kamera aufgenommenen Bildes kann daher das Reflexionsspektrum einer Pflanze 18 ermittelt werden. Bei der Ermittlung des Reflexionsspektrums können optional auch Informationen zum Emissionsspektrum der Beleuchtungseinheit 12 hinzugezogen werden. Die in der Fig. 2 dargestellte zweite Linse L2 dient dazu, dass vom Beugungsgitter G aufgefächerte und divergierende Licht erneut zu kollimieren, sodass die Strahlung die Sensorfläche der CMOS-Kamera erreicht.

In der Fig. 3 ist ein drittes Ausführungsbeispiel der erfindungsgemäßen Analysevorrichtung 10 dargestellt. Bei diesem Ausführungsbeispiel umfasst die Sensoreinheit 14 ein Spektrometer 14a zur Aufnahme spektraler Informationen, eine erste 3D-Kamera 14b zur Aufnahme der dreidimensionalen Kontur der Pflanze 18, eine zweite 3D-Kamera 14c, die der Aufnahme der dreidimensionalen Kontur der Pflanze 18 aus einer zusätzlichen Perspektive dient, und einen zusätzlichen Sensor 14d, der zur Aufnahme von Umweltdaten verwendet wird. Gemäß dem in der Fig. 3 dargestellten Ausführungsbeispiel kann die erste 3D-Kamera 14b derart angeordnet sein, dass sie die dreidimensionale Kontur der Vorderseite der Pflanze 18 (bzw. des Pflanzenblattes) aufnimmt, während die zweite 3D-Kamera 14c die dreidimensionale Kontur der Rückseite der Pflanze (bzw. des Pflanzenblattes) 18 erfasst. Auf diese Weise wird es ermöglicht, das Volumen der Pflanze 18 zu ermitteln und daraus beispielsweise die absolute THC-Konzentration zu berechnen. Der zusätzliche Sensor 14d kann beispielsweise die Lufttemperatur im Gewächshaus bzw. in der Klimakammer messen und zusätzliche Informationen für den Klassifikator bereitstellen.

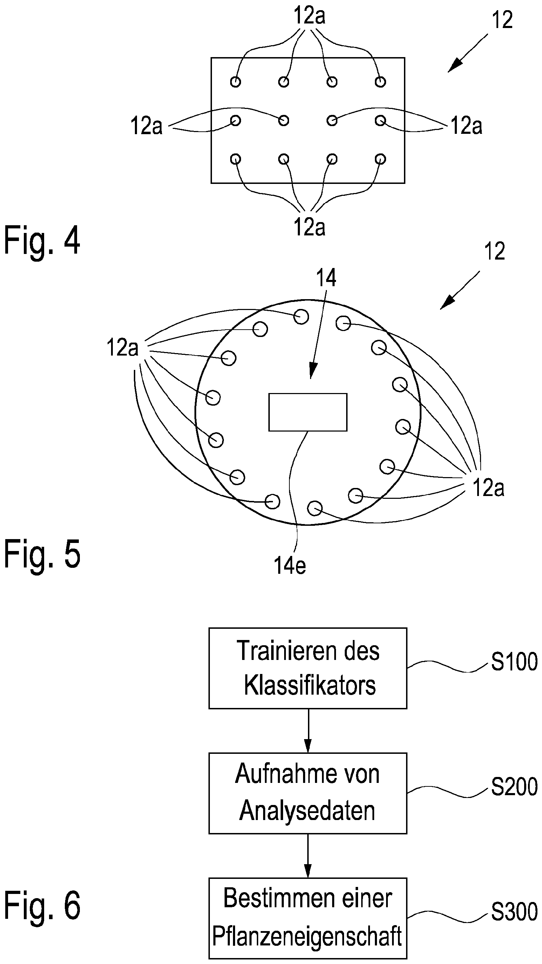

In der Fig. 4 ist eine Draufsicht auf ein erstes Ausführungsbeispiel der Beleuchtungseinheit 12 dargestellt. Wie in dieser Fig. zu erkennen ist, umfasst die Beleuchtungseinheit 12 insgesamt zwölf LEDs 12a. Bei den LEDs 12a kann es sich um identische LEDs oder aber auch um LEDs mit unterschiedlichen Emissionsspektren handeln. Dabei können schmalbandig emittierende LEDs zum Einsatz kommen oder aber auch breitbandige LEDs.
In der Fig. 5 ist eine Draufsicht auf ein zweites Ausführungsbeispiel der Beleuchtungseinheit 12 dargestellt. In diesem Ausführungsbeispiel ist die Sensoreinheit 14, die als Kamera ausgeführt ist und eine Sensorfläche 14e aufweist, in der Beleuchtungseinheit 12 integriert. Die einzelnen LEDs 12a sind dabei auf einer Kreisbahn angeordnet, welche die Sensorfläche 14e umgibt. Dadurch kann eine besonders homogene Ausleuchtung der zu analysierenden Pflanze 18 sowie eine besonders vorteilhafte Lichtausbeute der Kamera erreicht werden.

In der Fig. 6 ist eine Übersicht über das erfindungsgemäße Verfahren gegeben. Zunächst wird der Klassifikator, der insbesondere auf einem neuronalen Netz basiert, unter Verwendung eines Trainings-Datensatzes trainiert (Schritt S100). Dieser Trainings-Datensatz enthält spektrale Informationen, denen bestimmte Eigenschaften einer Pflanze 18 zuzuordnen sind. Beispielsweise kann der Klassifikator während der Trainingsphase lernen, dass ein Absorptionspeak bei 350 m darauf hinweist, dass die zu analysierenden Pflanze 18 von einer bestimmten Krankheit befallen ist. Der Klassifikator könnte während der Trainingsphase auch lernen, dass die Messung von zwei Absorptionspeaks, von denen das erste Peak bei 320 nm und das zweite Peak bei 480 nm detektiert wird, darauf hinweist, dass die Pflanze gesund ist und einen hohen Wassergehalt aufweist. Auch kann der Klassifikator dahingehend trainiert werden, dass dieser weitere Eingangsdaten berücksichtigt, insbesondere zusätzliche Bildaufnahmen oder Informationen über die Temperatur, Feuchtigkeit, Lichtverhältnisse, genetische Informationen der Pflanze und/oder die Pflanzensorten. Je mehr Trainingsdaten während der Trainingsphase vorhanden sind, desto zuverlässiger kann der Klassifikator zu einem späteren Zeitpunkt die zu analysierende Pflanze bewerten. Nach dem Trainieren des Klassifikators (Schritt S100) ist das erfindungsgemäße Verfahren einsatzbereit.

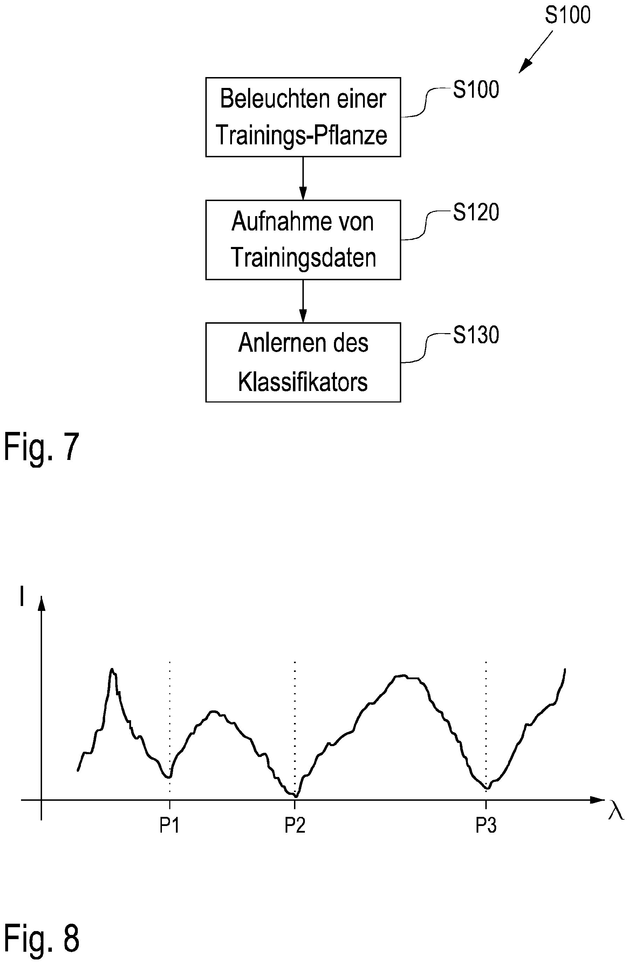

Um eine Pflanze zu analysieren, erfolgt zunächst eine Aufnahme der Analyse-Eingangsdaten (Schritt S200). Die Aufnahme der Analyse-Eingangsdaten erfolgt im Wesentlichen genauso wie bei der Aufnahme der Trainings-Eingangsdaten, worauf noch im Zusammenhang mit der Fig. 7 eingegangen wird. Sobald die Analyse-Eingangsdaten, insbesondere ein Absorptionsspektrum, aufgenommen sind, erfolgt die Bestimmung der Pflanzeneigenschaft (Schritt S300) mithilfe des zuvor trainierten Klassifikators.

In der Fig. 7 sind die einzelnen Unterschritte des Trainierens des Klassifikators (Schritt S100) dargestellt. Zunächst wird eine Trainings-Pflanze mit bekannten Eigenschaften mittels der Beleuchtungseinheit 12 beleuchtet (Schritt S110). Anschließend werden die Trainingsdaten, genauer gesagt die Trainings-Eingangsdaten, mittels der Sensoreinheit 14 aufgenommen (Schritt S120). Die Trainings-Eingangsdaten werden anschließend den vorgegebenen und bereits bekannten Trainings-Ausgangsdaten, die Informationen über die Eigenschaft einer Pflanze aufweisen, zugeordnet. Auf diese Weise "lernt" der Klassifikator, welche Ausgangsdaten bzw. Eigenschaften mit welchen Eingangsdaten korrelieren. Nach dem Anlernen des Klassifikators (Schritt S130) ist das Verfahren bzw. der Klassifikator einsatzbereit.

In der Fig. 8 ist beispielhaft ein aufgenommenes Reflexionsspektrum dargestellt. Dieses Reflexionsspektrum kann beispielsweise während des Analyseverfahrens aufgenommen worden sein. Das Reflexionsspektrum stellt das Ergebnis einer spektralen Analyse des von einer Pflanze reflektierten Lichtes dar. Bei dem dargestellten Reflexionsspektrum sind drei verschiedene Punkte mit P1, P2 und P3 gekennzeichnet. Bei diesen Punkten handelt es sich um lokale Minima des Reflexionsspektrums bzw. um lokale Maxima eines entsprechenden Absorptionsspektrums. Diese lokalen Extremwerte können beispielsweise in vorgegebenen Bereichen gesucht und ermittelt werden. Beispielsweise kann es vorgesehen sein, dass die Extremwerte in den Wellenlängenbereichen b1 = 200 bis 400 nm, b2 = 400 bis 600 nm und b3 = 600 bis 800 nm ermittelt werden. Das Ergebnis einer entsprechenden Analyse kann beispielswese sein, dass ein erster Absorptionspeak bei P1 = 280 nm, ein zweiter Absorptionspeak bei P2 = 550 nm und ein dritter Absorptionspeak bei P3 = 790 nm ermittelt wird. Diese drei Werte können also als Analyse-Eingangsdaten verwendet werden, um etwa zu bestimmen, dass die analysierte Pflanze der Sorte Sativa angehört und zudem gesund ist.

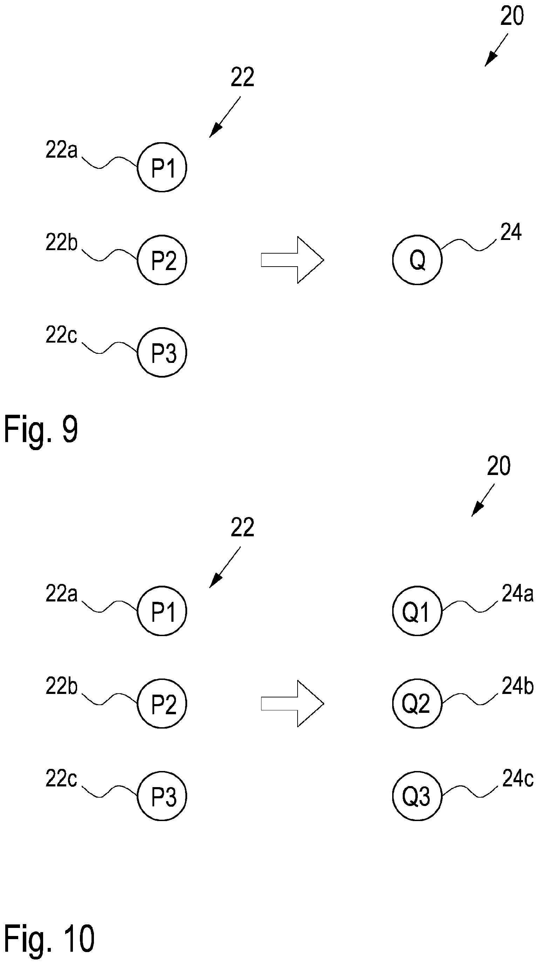

Die prinzipielle Funktionsweise der im Zusammenhang mit der Erfindung verwendeten Klassifikatoren ist in den Fign. 9 und 10 abstrahiert dargestellt.

In der Fig. 9 ist der Fall dargestelltt, dass bei der Analyse einer Pflanze die drei Merkmale P1, P2, und P3 ermittelt werden. Bei diesen Merkmalen kann es sich, wie vorstehend erläutert, beispielsweise um drei Absorptionspeaks handeln. In dem in der Fig. 9 dargestellten Beispiel werden die Merkmale P1 bis P3 als Eingangsdaten für den Klassifikator verwendet, um auf ein Ausgangsdatum Q zu schließen. Q kann beispielsweise den Zustand der Pflanze (z.B. krank oder gesund) oder die THC-Konzentration betreffen.

Schließlich ist in der Figur 10 der Fall dargestellt, bei dem zur Analyse einer Pflanze die drei Merkmale P1, P2 und P3 als Eingangsdaten verwendet werden und die Größen Q1, Q2 und Q3 die Ausgangsdaten darstellen. Wie bei den vorstehenden Beispielen erläutert, können P1 bis P3 drei lokale Absorptionspeaks beschreiben. Die drei Ausgangsgrößen Q1 bis Q3 hingegen können drei Eigenschaften einer Pflanze beschreiben. Beispielsweise kann Q1 die Pflanzensorte beschreiben, während Q2 beispielsweise den Gesundheitszustand einer Pflanze und Q3 den Wassergehalt der Pflanze beschreibt.

### Bezugszeichenliste

- 10: Analysevorrichtung
- 12: Beleuchtungseinheit
- 12a: LED
- 14: Sensoreinheit
- 14a: Spektrometer
- 14b: erste 3D-Kamera
- 14c: zweite 3D-Kamera
- 14d: Temperatursensor
- 14e: Sensorfläche
- 16: Auswerteeinheit
- 18: Pflanze
- 20: Klassifikator
- 22: Eingangsdaten
- 22a: erstes Eingangsdatum
- 22b: zweites Eingangsdatum
- 22c: drittes Eingangsdatum
- 24: Ausgangsdaten
- 24a: erstes Ausgangsdatum
- 24b: zweites Ausgangsdatum
- 24c: drittes Ausgangsdatum
- S100: Verfahrensschritt des Trainierens
- S110: Verfahrensschritt der Beleuchtung einer Trainings-Pflanze
- S120: Verfahrensschritt der Aufnahme von Trainingsdaten
- S130: Verfahrensschritt des Anlernens des Klassifikators
- S200: Verfahrensschritt der Aufnahme von Analysedaten
- S300: Verfahrensschritt der Bestimmung der Pflanzeneigenschaft

## Patentansprüche

1. Verfahren zur Analyse einer Pflanze (18), insbesondere zur Analyse von Cannabis, unter Verwendung einer Beleuchtungseinheit (12), einer Sensoreinheit (14) und einer Auswerteeinheit (16), wobei die Auswerteeinheit (16) einen datenbasierten Klassifikator (20) enthält und das Verfahren die nachfolgenden Schritte aufweist:
- Trainieren (S100) des Klassifikators (20), mit den folgenden Schritten:
- Beleuchten (S110) einer Trainings-Pflanze mit mindestens einer bekannten Eigenschaft unter Verwendung der Beleuchtungseinheit (12);
- Aufnehmen (S120) von Trainings-Eingangsdaten durch Messung der von der Trainings-Pflanze reflektierten Strahlung;
- Trainieren (S130) des Klassifikators (20) mit den aufgenommenen Trainings-Eingangsdaten sowie Trainings-Ausgangsdaten, wobei die Trainings-Eingangsdaten zumindest spektrale Informationen, insbesondere ein Absorptionsspektrum oder ein Reflexionsspektrum der Trainings-Pflanze, enthalten und die Trainings-Ausgangsdaten den Trainings-Eingangsdaten zugeordnet sind und Informationen über mindestens eine Eigenschaft der Trainings-Pflanze aufweisen;
- Aufnehmen (S200) von Analyse-Eingangsdaten mittels der Sensoreinheit (14), mit den folgenden Schritten:
- Beleuchten einer zu analysierenden Pflanze mit mindestens einer unbekannten Eigenschaft unter Verwendung der Beleuchtungseinheit (12);
- Aufnehmen von Analyse-Eingangsdaten durch Messung der von der zu analysierenden Pflanze reflektierten Strahlung;
- Bestimmen (S300) einer Eigenschaft der zu untersuchenden Pflanze unter Verwendung des zuvor mit den Trainings-Eingangsdaten und Trainings-Ausgangsdaten trainierten Klassifikators (20) und der aufgenommenen Analyse-Eingangsdaten.

2. Verfahren nach Anspruch 1, **dadurch gekennzeichnet, dass** zur Erhöhung der Genauigkeit des Verfahrens die Trainings-Eingangsdaten und die Analyse-Eingangsdaten zusätzlich Bildaufnahmen der Trainings-Pflanze und der zu analysierenden Pflanze enthalten.

3. Verfahren nach Anspruch 2, wobei das Trainieren des Klassifikators (20) zusätzlich folgende Schritte aufweist:
- Bereitstellen von Bildaufnahmen als Trainings-Eingangsdaten, die für das Trainieren des Klassifikators (20) verwendet werden sollen und von Trainings-Ausgangsdaten, die den bereitgestellten Bildaufnahmen zugeordnet sind;
- Rotieren der bereitgestellten Bildaufnahmen;
- Zuordnen der Trainings-Ausgangsdaten, die den ursprünglich bereitgestellten Bildaufnahmen zugeordnet sind, zu den rotierten Bildaufnahmen;
- Zusammenfügen der ursprünglich bereitgestellten Bildaufnahmen und der rotierten Bildaufnahmen sowie der den Bildaufnahmen zugeordneten Trainings-Ausgangsdaten zu einem erweiterten Trainings-Datensatz; und
- Trainieren des Klassifikators (20) unter Verwendung des erweiterten Trainings-Datensatzes.

4. Verfahren nach einem der Ansprüche 1 bis 3, **dadurch gekennzeichnet, dass** zur Erhöhung der Genauigkeit des Verfahrens die Trainings-Eingangsdaten und die Analyse-Eingangsdaten zusätzlich genetische Informationen von Pflanzen (18) enthalten.

5. Verfahren nach einem der Ansprüche 1 bis 4, **dadurch gekennzeichnet, dass** der Klassifikator (20) auf einem künstlichen neuronalen Netz, insbesondere auf einem Convolutional Neural Network, basiert.

6. Verfahren nach einem der Ansprüche 1 bis 5, **dadurch gekennzeichnet, dass** zur Erhöhung der Genauigkeit des Verfahrens beim Trainieren des Klassifikators (20) die Trainings-Eingangsdaten und die Analyse-Eingangsdaten Informationen über die zeitliche Änderung der aufgenommenen Eingangsdaten aufweisen.

7. Vorrichtung zur Analyse einer Pflanze (18), insbesondere einer Hanfpflanze, umfassend:
- eine Beleuchtungseinheit (12) zur Beleuchtung der zu analysierenden Pflanze;
- eine Sensoreinheit (14) zur Aufnahme von Analyse-Eingangsdaten, wobei die Analyse-Eingangsdaten zumindest spektrale Informationen, insbesondere ein Absorptionsspektrum oder ein Reflexionsspektrum der Trainings-Pflanze, enthalten; und
- eine Auswerteeinheit (16) zur Auswertung der aufgenommenen Analyse-Eingangsdaten und zum Bestimmen mindestens einer Eigenschaft der zu analysierenden Pflanze; wobei
- die Auswerteeinheit (16) dazu ausgelegt ist, die mindestens eine Eigenschaft der Pflanze unter Verwendung eines datenbasierten Klassifikators (20) sowie der zuvor aufgenommenen Analyse-Eingangsdaten zu bestimmen.

8. Vorrichtung nach Anspruch 7, **dadurch gekennzeichnet, dass** die Sensoreinheit (14) ein Spektrometer (14a) aufweist.

9. Vorrichtung nach einem der Ansprüche 7 oder 8, **dadurch gekennzeichnet, dass** die Sensoreinheit (14) eine Kamera, insbesondere eine CMOS-Kamera, umfassend eine Sensorfläche (14e) aufweist.

10. Vorrichtung nach Anspruch 9, **dadurch gekennzeichnet, dass** die Kamera als 3D-Kamera (14b, 14c), insbesondere als Stereokamera, ausgeführt ist.

11. Vorrichtung nach einem der Ansprüche 7 bis 10, **dadurch gekennzeichnet, dass** die Beleuchtungseinheit (12) mindestens zwei Beleuchtungselemente, insbesondere mindestens zwei LEDs (12a), aufweist.

12. Vorrichtung nach einem der Ansprüche 7 bis 11, **dadurch gekennzeichnet, dass** die Beleuchtungselemente auf einer Kreisbahn angeordnet sind, die die Sensorfläche (14e) der Kamera umgibt.

13. Vorrichtung nach einem der Ansprüche 7 bis 12, **dadurch gekennzeichnet, dass** die Sensoreinheit (14) und/oder die Beleuchtungseinheit (12) ein Kühlelement aufweisen.

14. Vorrichtung nach einem der Ansprüche 7 bis 13, **dadurch gekennzeichnet, dass** die Sensoreinheit (14) zusätzlich einen Temperatursensor und/oder einen Feuchtigkeitssensor aufweist.

15. Vorrichtung nach einem der Ansprüche 7 bis 14, **dadurch gekennzeichnet, dass** die Sensoreinheit (14) eine mit einer UV-zu-VIS Umwandlungsbeschichtung versehene Sensorfläche (14e) aufweist.
